# EUROPÄISCHE PATENTSCHRIFT

(11) **EP 2 251 682 B1**
(45) Veröffentlichungstag und Bekanntmachung des Hinweises auf die Patenterteilung: **14.11.2012**
(21) Anmeldenummer: 09160273.0
(22) Anmeldetag: 14.05.2009
(51) Int. Cl.: G01N 27/38, G01N 27/403, G01N 33/18

(54) **Wasseranalyse-Tauchsonde mit einer reinigbaren Elektrode zur Bestimmung eines Analyts in Wasser**
Water analysis submersible probe with a cleanable electrode for determining an analyte in water
Sonde d'immersion pour l'analyse de l'eau dotée d'une électrode nettoyable destinée à la détermination d'un analyte dans l'eau

(43) Veröffentlichungstag der Anmeldung: 17.11.2010
(73) Patentinhaber: Hach Lange GmbH, 14163 Berlin (DE)
(72) Erfinder: Leyer, Axel, 41199, Mönchengladbach (DE); Heidemanns, Lothar, 41352, Korschenbroich (DE); Jonak, Andreas, 40667, Meerbusch (DE); Dr. Hahn, Markus, 47906, Kempen (DE); Kussmann, Michael, 40476, Düsseldorf (DE); Rudde, Heinz, 41836, Hückelhoven (DE); Rieger, Claudia, 40227, Düsseldorf (DE)
(74) Vertreter: ter Smitten, Hans

(56) Entgegenhaltungen:
- EP-A- 1 710 567
- JP-A- 2005 211 858
- JP-A- 2006 322 793
- JP-U- 53 103 786
- JP-U- 55 018 148
- US-A- 4 138 638

## Beschreibung

Die Erfindung bezieht sich auf eine Wasseranalyse-Tauchsonde mit einer Elektrode zur Bestimmung eines Analyts in Wasser.

Wasseranalyse-Tauchsonde werden zur Prozessanalyse bei der Abwasser-Klärung, der Trinkwasser-Kontrolle etc. eingesetzt, um einen oder mehrere bestimmte Analyte in dem Wasser quasi-kontinuierlich zu bestimmen. Die Tauchsonde weist hierzu stirnseitig eine oder mehrere Elektroden auf, die zu Fouling und Verzopfung neigen können. Dem Fouling und der Verzopfung kann vorgebeugt werden, indem sie Stirnseite der Tauchsonde mit Luft gespült wird. Tauchsonden mit Luftspülung nach dem Stand der Technik sind relativ komplex aufgebaut, so dass der Auseinanderbau und Zusammenbau der Tauchsonde für Wartung und Reparatur aufwändig sind.

JP 2006 322793 A offenbart eine Sonde, die einen Deckel aufweist, der die Stirnseite mit den Elektroden des Sondenkörpers vollständig abschirmt. Durch Filter verschlossene Öffnungen sind seitlich vorgesehen.

Aus JP 53 103786 U Ist eine Sonde mit einem Deckel bekannt, der keine Elektrodenöffnungen aufweist.

JP 55 018148 U offenbart eine Laborsonde mit einem Deckel, der nicht entfernbar ist.

US 4138 638 offenbart eine weit von der Elektrode entfernte Öffnung. Die Elektrode wird durch den Deckel abgeschirmt. Der Deckel ist nicht abziehbar.

EP 1 710 567 offenbart eine Abwaneranalyse sensor Kartushe für eine Tanksonde.

Aus JP 2005/211858 ist eine Sonde bekannt, bei der die Elektroden über vertikale Luftleitungen, die den Sondenkörper umgeben, radial mit Luft gespült werden.

Aufgabe der Erfindung ist es demgegenüber, eine luftgespülte Tauchsonde mit verzögerungsfreier Analytbestimmung und mit einem vereinfachten Auseinander- und Zusammenbau zu schaffen.

Diese Aufgabe wird erfindungsgemäß gelöst mit den Merkmalen des Patentanspruches 1.

Die erfindungsgemäße Wasseranalyse-Tauchsonde weist an der Stirnseite ihres Sondenkopfes eine Elektrode auf. An dem Sondenkopf ist eine in distaler Richtung abziehbare separate Schutzkappe befestigt, die an ihrer Stirnseite eine Elektrodenöffnung und eine Luftaustrittsdüse aufweist. Die Luftaustrittsdüse ist derart orientiert, dass die hieraus austretende Luft über die Elektrode streicht. Die Luftaustrittsdüse ist beispielsweise auf die Elektrode gerichtet.

Die Schutzkappe hat im weitesten Sinne auch eine mechanisch schützende Funktion für den Sondenkopf und umgibt den Sondenkopf seitlich und teilweise auch auf der Stirnseite. Auf der Stirnseite weist die Schutzkappe eine Elektrodenöffnung für eine oder mehrere Elektroden auf, die in unmittelbarem Kontakt mit dem Wasser stehen, und nicht durch die Schutzkappe abgedeckt sind. Allerdings können die Elektroden in der Elektrodenöffnung versenkt angeordnet sein. Der Sondenkopf und insbesondere die Elektroden sind durch die Schutzkappe geschützt vor in dem Wasser herum treibenden in Gegenständen. Die Schutzkappe kann distal von dem Sondenkopf abgezogen werden, das heißt, bei in dem Wasser nach unten hängender Tauchsonde nach unten abgezogen werden. Die Luftaustrittsdüse verbleibt dabei an der Schutzkappe. Die Luftaustrittsdüse ist mit einer einfachen Kupplung axial mit der Spülluft-Versorgung gekoppelt. Ferner ist die Schutzkappe bevorzugt mit einem einfach zu lösenden und zu schließenden Mittel lösbar an dem Sondenkopf oder dem Sondenkörper fixiert.

Erfindungsgemäss weist die Schutzkappe einen zirkulären Luft-Ringkanal und einen Luft-Axialkanal auf, der Spülluft aus dem Ringkanal in die Luftaustrittsdüse leitet. Der Ringkanal vor allem in die Funktionen, die Spülluft über den gesamten Umfang der Schutzkappe zu verteilen, um mehrerer Luft-Axialkanäle, die über den Umfang verteilt sind, auf diese Weise mit Spülluft zu versorgen. Zum anderen kann der Ringkanal auch eine Luft-Einlassöffnung aufweisen, in die eine entsprechende Luft-Versorgungsleitung mündet. Das Volumen des Ringkanals und der Luft-Axialkanäle sollte insgesamt möglichst klein sein, damit das Wasser, das bei in das Wasser eingetauchter Tauchsonde einen überatmosphärischen Druck aufweist, möglichst wenig tief in die Luft-Axialkanäle eindringt. Das geringe Gesamtvolumen wird dadurch realisiert, dass lediglich ein möglichst kleiner Ringkanal vorgesehen ist, und er axiale Transport der Spülluft durch sehr kleine Axialkanäle, beispielsweise durch Kapillaren, realisiert wird.

Erfindungsgemäss wird der Ringkanal Roxy mal von einer Außenwand des Sondenkopfes und distal von der Schutzkappe gebildet. Die Schutzkappe selbst weist also einen proximal offenen Ringkanal auf, dessen proximale Öffnung erst durch die Außenwand des Sondenkopfes geschlossen wird. Durch den Verzicht auf einen in sich geschlossenen Ringkanal in der Schutzkappe kann eine relativ einfache Herstellung der Schutzkappe sichergestellt werden.

Vorzugsweise wird der Luft-Axialkanal von einem separaten Luftleitungsrohr gebildet, das in den Schutzkappen-Körper axial eingesteckt ist. Der Schutzkappen-Körper weist hierzu lediglich eine bis zu dem Ringkanal durchgehende axiale Bohrung auf, in die das Luftleitungsrohr axial eingesetzt ist. Das Luftleitungsrohr kann an seinem distalen Ende einen Bogen von 80 bis 120° aufweisen, durch den die Luft schließlich in ungefähr radialer Richtung austritt. Vorzugsweise besteht das Luftleitungsrohr aus Edelstahl.

Gemäß einer bevorzugten Ausgestaltung wird der Sondenkopf von einer separaten austauschbaren Sensorkartusche gebildet. Die Sensorkartusche enthält die Elektroden, die in der Regel Verschleiß ausgesetzt sind, und daher regelmäßig ausgetauscht werden müssen. Ferner kann die Sensorkartusche ein Elektrolyt in einem Tank aufweisen, das nach gewisser Zeit ebenfalls erschöpft ist. Durch Austausch der Sensorkartusche werden in einem einzigen Arbeitsgang alle Elektroden und alle übrigen Verbrauchsmaterialien ausgetauscht. Vor dem Austausch der Sensorkartusche muss lediglich die Schutzkappe von der Sensorkartusche abgezogen werden, und nach dem Aufsetzen der neuen Sensorkartusche die Schutzkappe einfach wieder auf die Sensorkartusche aufgesetzt werden.

Gemäß einer bevorzugten Ausführungsform weist die Schutzkappe mindestens zwei Rastlaschen zur rastenden Befestigung an dem Tauchsonden-Körper auf. Die Rastlaschen sind grundsätzlich völlig ausreichend, um den Sondenkopf an dem Tauchsonden-Körper zu befestigen. Ferner sind die Rastlaschen bei Wartung beziehungsweise Reparatur der Tauchsonde sehr einfache manuell zu öffnen, um die Schutzkappe abzuziehen. Das Entfernen beziehungsweise fixieren der Schutzkappe kann auf diese Weise ohne jedes Werkzeug durchgeführt werden.

Vorzugsweise weist die Elektrode eine Membran, beispielsweise eine ionenselektive Membran an der Stirnseite auf. Derartige Membranen sind in Wasser, insbesondere in Abwasser, Verschleiß ausgesetzt, und müssen daher durch ständige Spülung mit Spülluft von Fouling-Bewuchs freigehalten werden.

Gemäß einer bevorzugten Ausgestaltung wird die Schutzkappe von einem einstückigen Schutzkappen-Körper gebildet, der besonders bevorzugt aus einem gummielastischen Kunststoff besteht. Durch den gummielastischen Kunststoff-Körper ist einerseits ein besonders wirksamer mechanischer Schutz des Sondenkopfes gegen kollidierende Gegenstände gewährleistet. Ferner kann durch die Elastizität des Schutzkappen-Körpers ein einfacher Verschluss, beispielsweise in Form von zwei oder mehr Rastlaschen realisiert werden.

Im Folgenden wird unter Bezugnahme auf die Zeichnungen ein Ausführungsbeispiel der Erfindung näher erläutert.

Es zeigen:
Figur 1 einen Längsschnitt durch einen Sondenkopf einer Wasseranalyse-Tauchsonde,
Figur 2 eine Draufsicht auf die Stirnseite des Sondenkopfes in der Figur 1, und
Figur 3 eine perspektivische Ansicht des Sondenkopfes der Figur 1 von hinten.

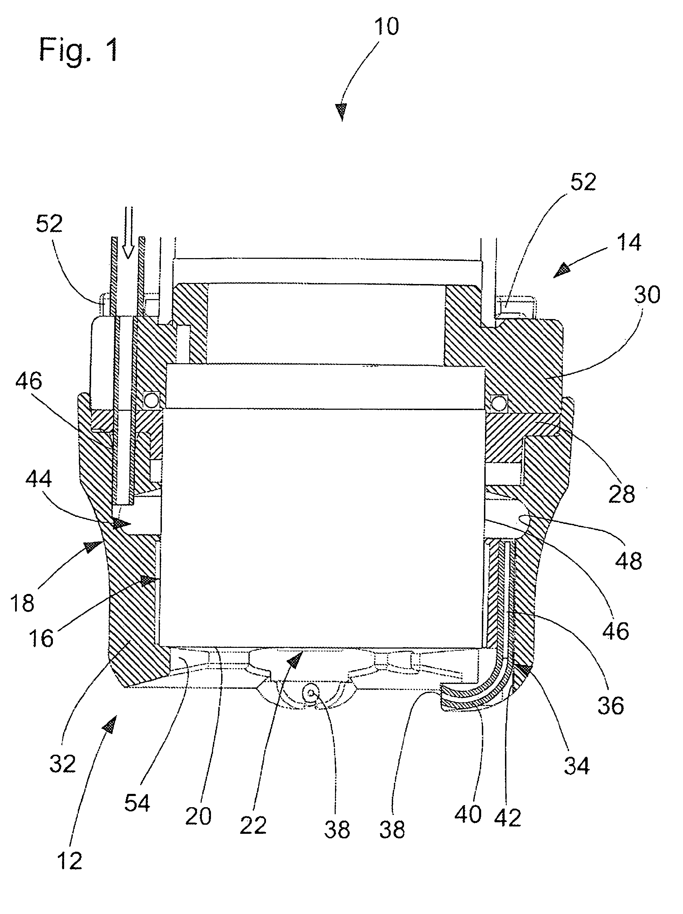

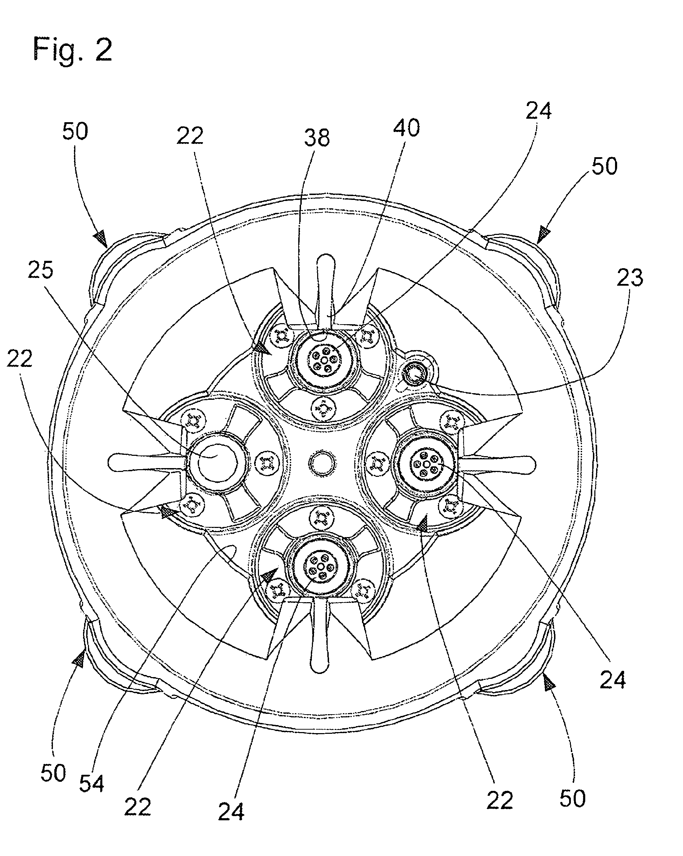

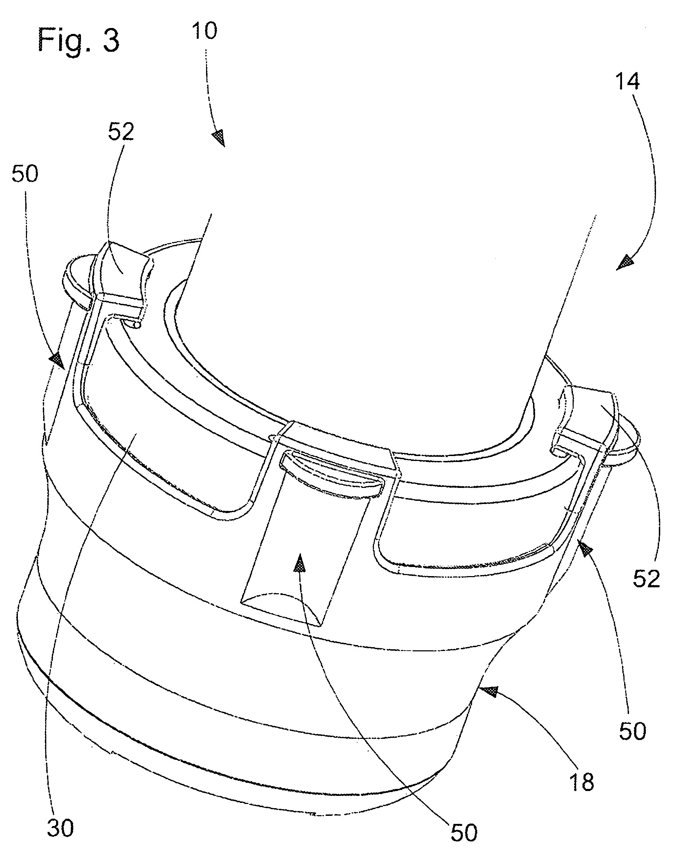

In den Figuren 1 bis 3 ist einer Wasseranalyse-Tauchsonde 10 mit einem Sondenkopf 12 dargestellt. Die Tauchsonde 10 dient der quantitativen Bestimmung von drei verschiedenen Analyten in Abwasser. Die Tauchsonde 10 weist im wesentlichen einen Tauchsonden-Körper 14, eine daran befestigte Sensorkartusche 16 und eine die Sensorkartusche 16 umfassende Schutzkappe 18 auf.

Die Sensorkartusche 16 weist auf ihrer Stirnseite 20 vier Elektroden 22 sowie eine so genannte Salzbrücke 23 auf. Ein wesentliches Element der Elektroden 22 sowie der Salzbrücke 23 sind ihre jeweiligen Membranen 24, 25 bzw. messtechnisch sensitiven Oberflächen die empfindlich gegen Fouling-Bewuchs und Verzopfung sind. Die Sensorkartusche 16 weist innenseitig einen Tank für ein Elektrolyt auf. Die Sensorkartusche 16 weist einen umlaufenden Flansch 28 auf, der mit einem entsprechenden umlaufenden Flansch 30 des Tauchsonden-Körpers 14 mit geeigneten Befestigungsmitteln, beispielsweise Schrauben, verbunden ist. Die Sensorkartusche 16 kann durch Lösen der Schrauben von dem Tauchsonden-Körper 14 gelöst werden und weist elektrische Stecker auf, die mit entsprechenden elektrischen Steckern des Tauchsonden-Körpers 14 lösbar zusammengesteckt sind. Die Sensorkartusche 16 ist austauschbar ausgebildet.

Die Schutzkappe 18 besteht im wesentlichen aus einem einstückigen Schutzkappen-Körper 32 aus gummielastischem Kunststoff, der distal eine Elektrodenöffnung 54 an der Stirnseite aufweist, und weist ferner vier Edelstahl-Luftleitungsrohre 34 auf, die jeweils einen Luft-Axialkanal 36 bilden und eine Luftaustrittsdüse 38 aufweisen. Die Luftleitungsrohre 34 weisen distal jeweils einen Bogen 40 von circa 110° auf, durch den die Luftaustrittsdüse 38 eine radiale und leicht proximale Orientierung erhält. Jede der vier Luftaustrittsdüsen 38 ist jeweils einer Elektrode 22 beziehungsweise der Salzbrücke 23 zugeordnet und genau auf diese gerichtet, so dass die durch die Luftaustrittsdüse 38 austretende Luft über die betreffende Elektrode 22 streicht.

Die vier Luftleitungsrohre 34 stecken jeweils in axialen Bohrungen 42 des Schutzkappen-Körpers 32, die in einen geschlossenen umlaufenden Ringkanal 44 münden. Der Ringkanal 44 wird durch eine LuftversorgungsLeitung 46 mit Luft versorgt, die außen an dem Tauchsonden-Körper 16 axial verlegt ist. Der Ringkanal 44 wird radial proximal, also innenseitig, von einer Außenwand 46 der Sensorkartusche 16 und distal hiervon von einer radial innenseitig offenen Ringnut 48 in dem Schutzkappen-Körper 32 gebildet.

Die Schutzkappe 18 weist vorliegend vier elastische Rastlaschen 50 mit Rastkörper 52 auf, die in der in den Figuren 1-3 gezeigten Verrastungsposition den Flansch 30 des Tauchsonden-Körpers 14 hintergreifen.

Zum Auswechseln der Sensorkartusche 16 werden die Rastlaschen 50 manuell nach außen gebogen und die Schutzkappe 18 axial distal abgezogen. Anschließend kann die Sensorkartusche 16 nach Lösen der entsprechenden Befestigungsmittel von dem Tauchsonden-Körper gelöst werden. Dann kann eine neue Sensorkartusche 16 an dem Tauchsonden-Körper 16 mit Hilfe der Befestigungsmittel wieder befestigt werden. Schließlich wird die Schutzkappe 18 axial wieder auf die Sensorkartusche 16 aufgeschoben, wobei in der Verrastungsposition die vier elastischen Rastlaschen 50 mit dem Flansch 30 des Tauchsonden-Körpers 14 verrasten und die Schutzkappe 18 an dem Tauchsonden-Körper 14 festhalten.

## Patentansprüche

1. Wasseranalyse-Tauchsonde (10) mit mindestens einer Elektrode (22) zur Bestimmung eines Analyts in Wasser, mit
einem Sondenkopf (12) mit einer Stirnseite (20), an der die Elektrode (22) angeordnet ist, und
einer an dem Sondenkopf (12) in distaler Richtung abziehbar befestigten separaten Schutzkappe (18), die stirnseitig eine Elektrodenöffung (54) für eine oder mehrere Elektroden (22) aufweist, wobei
die eine oder mehrere Elektroden (22) nicht durch die Schutzkappe (18) abgedeckt sind,
die Schutzkappe (18) eine Luftaustrittsdüse (38) aufweist, die derart orientiert ist, dass die durch die Luftaustrittsdüse (38) austretende Luft über die Elektrode (22) streicht,
die Schutzkappe (18) einen Luft-Ringkanal (44) und einen Luft-Axialkanal (36) aufweist, der Luft aus dem Ringkanal (44) in die Luftaustrittsdüse (38) leitet,
der Ringkanal (44) eine Lufteinlassöffnung aufweist, in die eine Luftversorgungsleitung mündet, und
der Ringkanal (44) proximal von einer Außenwand (46) des Sondenkopfs (12) und distal von der Schutzkappe (18) gebildet wird.

2. Wasseranalyse-Tauchsonde (10) nach Anspruch 1, wobei der Axialkanal (36) von einem separaten Luftleitungsrohr (34) gebildet wird.

3. Wasseranalyse-Tauchsonde (10) nach einem der vorangegangenen Ansprüche, wobei das Luftleitungsrohr (34) aus Edelstahl besteht.

4. Wasseranalyse-Tauchsonde (10) nach einem der vorangegangenen Ansprüche, wobei der Sondenkopf (12) von einer separaten austauschbaren Sensorkartusche (16) gebildet wird.

5. Wasseranalyse-Tauchsonde (10) nach einem der vorangegangenen Ansprüche, wobei die Schutzkappe (18) mindestens zwei Rastlaschen (50) zur rastenden Befestigung an einem Tauchsonden-Körper (14) aufweist.

6. Wasseranalyse-Tauchsonde (10) nach einem der vorangegangenen Ansprüche, wobei die Elektrode (22) eine Membran (24) an der Stirnseite (20) aufweist.

7. Wasseranalyse-Tauchsonde (10) nach einem der vorangegangenen Ansprüche, wobei die Schutzkappe (18) von einem einstückigen Schutzkappen-Körper (32) gebildet wird.

8. wasseranalyse-Tauchsonde (10) nach einem der vorangegangenen Ansprüche, wobei die Schutzkappe (18) im Wesentlichen aus gummielastischem Kunststoff besteht.

## Claims

1. Water analysis submersible probe (10) with at least one electrode (22) for determining an analyte in water, comprising
a probe head (12) with a front end face (20) at which the electrode (23) is arranged, and
a separate protective cap (18) mounted to the probe head (12) such that it can be pulled off therefrom in the distal direction, the front end of said cap having an electrode opening (54) therein for one or a plurality of electrodes (23), wherein
said one or said plurality of electrodes (22) are not covered by the protective cap (18),
said protective cap (18) has an air outlet nozzle (38) oriented such that the air leaving the air outlet electrode (38) flows over the electrode (22),
the protective cap (18) comprises an annular air channel (44) and an axial air channel (36) leading air form the axial channel (44) into the air outlet nozzle (38),
the annular channel (44) has an air inlet opening into which an air supply line opens, and
the annular channel (44) is formed proximally by an outer wall (46) of the probe head (12) and is formed distally by the protective cap (18).

2. Water analysis submersible probe (10) of claim 1, wherein the axial channel (36) is formed by a separate air conduit (24).

3. Water analysis submersible probe (10) of one of the preceding claims, wherein the air conduit (34) is made of stainless steel.

4. Water analysis submersible probe (10) of one of the preceding claims, wherein the probe head (12) is formed by a separate exchangeable sensor cartridge (16).

5. Water analysis submersible probe (10) of one of the preceding claims, wherein the protective cap (18) has at least two locking tabs (50) for fastening the same to a submersible probe body (14) in a locking manner.

6. Water analysis submersible probe (10) of one of the preceding claims, wherein the electrode (22) has a membrane (24) on the front face side (20).

7. Water analysis submersible probe (10) of one of the preceding claims, wherein the protective cap (18) is formed by an integrally formed protective cap body (32).

8. Water analysis submersible probe (10) of one of the preceding claims, wherein the protective cap (18) is substantially made of rubber-elastic plastic material.

## Revendications

1. Sonde d'immersion (10) pour l'analyse de l'eau dotée d'au moins une électrode (22) destinée à la détermination d'un analyte dans l'eau, comprenant
une tête de sonde (12) avec une face de front (20) où est disposée ladite électrode (22), et
un capuchon protecteur (18) séparé, monté à ladite tête de sonde (12) de facon retirable dans la direction distale, ledit capuchon comprenant dans son coté de front une ouverture d'électrode (54) pour une ou plusieurs électrodes (22),
ladite une ou lesdites plusieurs électrodes (22) n'étant pas recouvertes par ledit capuchon protecteur (18),
ledit capuchon protecteur (18) comprenant une buse de sortie d'air (38) orientée tellement que l'air sortant de ladite buse de sortie d'air (38) coule sur ladite électrode (22),
ledit capuchon protecteur (18) comprenant un canal d'air annulaire (44) et un canal d'air axial (36) conduisant l'air du canal annulaire (44) dans ladite buse de sortie d'air (38),
ledit canal annulaire (44) comprenant une ouverture d'entrée d'air dans laquelle débouche un conduit d'alimentation en air, et
ledit canal annulaire (44) étant formé, coté proximal, par une paroi extérieure (46) de la tête de sonde (12) et, coté distal, par ledit capuchon protecteur (18).

2. Sonde d'immersion (10) pour l'analyse de l'eau selon la revendication 1, dans laquelle ledit canal axial (36) est formé par un tuyau d'air (34) séparé.

3. Sonde d'immersion (10) pour l'analyse de l'eau selon l'une quelconque des revendications précédentes, dans laquelle ledit tuyau d'air (34) est acier inoxydable.

4. Sonde d'immersion (10) pour l'analyse de l'eau selon l'une quelconque des revendications précédentes, dans laquelle ladite tête de sonde (12) est formée par une cartouche à capteur (16) séparée échangeable.

5. Sonde d'immersion (10) pour l'analyse de l'eau selon l'une quelconque des revendications précédentes, dans laquelle ledit capuchon protecteur (18) comprend au moins deux ergots de verrouillage (50) pour la fixation per verrouillage sur un corps de sonde d'immersion (14).

6. Sonde d'immersion (10) pour l'analyse de l'eau selon l'une quelconque des revendications précédentes, dans laquelle ladite électrode (22) comprend une membrane (24) sur la face de front (20).

7. Sonde d'immersion (10) pour l'analyse de l'eau selon l'une quelconque des revendications précédentes, dans laquelle ledit capuchon protecteur (18) est formé par un corps de capuchon protecteur (32) intégral.

8. Sonde d'immersion (10) pour l'analyse de l'eau selon l'une quelconque des revendications précédentes, dans laquelle ledit corps protecteur (18) est sensiblement formé d'une matière plastique avec élasticité de caoutchouc.
